# EUROPEAN PATENT SPECIFICATION

(11) **EP 3 593 823 B1**
(45) Date of publication and mention of the grant of the patent: **22.07.2026**
(21) Application number: 18764330.9
(22) Date of filing: 08.03.2018
(51) Int. Cl.: A61K 49/18, A61B 5/055, G01R 33/34

(54) **COMPOSITION FOR REMOVING NOISE FROM MRI AND PAD USING SAME**
ZUSAMMENSETZUNG ZUR RAUSCHENTFERNUNG AUS MRT UND PAD DAMIT
COMPOSITION POUR ÉLIMINER LE BRUIT D'UNE IRM ET TAMPON L'UTILISANT

(30) Priority: 09.03.2017 KR 20170030077; 16.05.2017 KR 20170060364
(43) Date of publication of application: 15.01.2020
(73) Proprietor: Baek, Jung-Eun, Seoul 04374 (KR)
(72) Inventor: PARK, Kyung Seok, Goyang-si Gyeonggi-do 10371 (KR)
(74) Representative: Dehns
(86) International application number: PCT/KR2018/002757
(87) International publication number: WO 2018/164502

(56) References cited:
- EP-A1- 2 932 983
- JP-B2- 3 163 570
- KR-A- 20130 085 386
- KR-A- 20140 109 420
- KR-B1- 101 131 362
- US-A1- 2011 152 670
- US-B2- 7 015 009
- US-B2- 8 644 906
- AFSHARIPOUR BABAK ET AL: "Using surface electromyography to detect changes in innervation zones pattern after human cervical spinal cord injury", 2016 38TH ANNUAL INTERNATIONAL CONFERENCE OF THE IEEE ENGINEERING IN MEDICINE AND BIOLOGY SOCIETY (EMBC), IEEE, 16 August 2016 (2016-08-16), pages 3757 - 3760, XP032979987, [retrieved on 20161013], DOI: 10.1109/EMBC.2016.7591545
- FRANKE A E ET AL: "POLYCRYSTALLINE SILICON-GERMANIUM FILMS FOR INTEGRATED MICROSYSTEMS", JOURNAL OF MICROELECTROMECHANICAL SYSTEMS, IEEE SERVICE CENTER, US, vol. 12, no. 2, 1 April 2003 (2003-04-01), pages 160 - 171, XP001181190, ISSN: 1057-7157, DOI: 10.1109/JMEMS.2002.805051
- PATEL SEJAL ET AL: "Pharmacokinetics of amitriptyline in rabbit skin and plasma following iontophoretic administrations", JOURNAL DRUG DEVELOPMENT AND INDUSTRIAL PHARMACY, vol. 36, no. 4, 6 April 2010 (2010-04-06), US, pages 379 - 384, XP093100262, ISSN: 0363-9045, DOI: 10.3109/03639040903188463
- W M TEEUWISSE ET AL: "New barium titanate based dielectric materials for high field imaging", PROC. INTL. SOC. MAG. RESON. MED., vol. 19, 2011, pages 622, XP055743512, Retrieved from the Internet <URL:https://cds.ismrm.org/protected/11MProceedings/files/622.pdf> [retrieved on 20201026]
- BEI ZHANG, ZAHI A FAYAD, JUNQIAN XU, BERND STOECKEL, PRITI BALCHANDANI: "Evaluating the SNR performance of using dielectric pads with multiple channel RF coils at 7T", PROC. INTL. SOC. MAG. RESON. MED., vol. 23, 15 May 2015 (2015-05-15), pages 1782, XP040667459
- KYUNG-SEOK PARK ET AL: "Development of New Materials for the Reduction of the Magnetic Resonance Imaging Metal Artifacts and the Improvement of the Image Quality", JOURNAL OF MAGNETICS, vol. 22, no. 3, 30 September 2017 (2017-09-30), pages 508 - 513, XP055580136, ISSN: 1226-1750, DOI: 10.4283/JMAG.2017.22.3.508

## Description

### [Technical Field]

The present invention relates to a method of removing noise from an **MRI** image using a pad as defined in the claims. The invention also relates to the use of the pad for removing noise from an **MRI** image. The pad used in the invention comprises a composition which includes one or more compounds having a functional group capable of hydrogen bonding.

### [Background Art]

A common **MRI** contrast agent is injected into the human body by an invasive procedure to enhance a signal according to the resonance of the nucleus of a hydrogen atom in the body, and thus obtain a clearer image of the core part of the body. However, the injection of a contrast agent by an invasive procedure, for example, intravenous injection, causes mild symptoms that need no special treatment, such as vomiting, abdominal pain, diarrhea, high blood pressure, headache, fever, chills, coughing, nasal congestion, etc., and also serious side effects such as dizziness, low blood pressure, bradycardia, systemic urticaria, angioedema, dyspnea, nephrogenic fibrosing dermopathy, nephrogenic systemic fibrosis (NSF), etc., and if there is a material affecting a magnetic field, such as a metal implant in the body, there is a limitation in that noise generated in an image is not removed.

In addition, the technique of removing noise due to a metal implant imbedded in the body, which is not able to be solved by a contrast agent, is mainly to estimate and compensate the defective part of the image using a computer algorithm, and this technique has problems of inaccuracy and insufficiently wide reproducible part. To solve the above-described problems, while much research is progressing to solve metal material noise and non-uniform distorted images, which are generated in the process of obtaining human body images, such that normal tissue and diseased tissue are more clearly distinguished using an MRI image (Korean Patent No. 10-1262479), there is less research on image enhancement technology that can improve both the limitation of image enhancement by software and the side effect of a contrast agent which is generated in the body. Particularly, no technology using a non-invasive composition, which is not injected into the body, in order to improve the above-described problems has been reported yet.

The inventors have intensively attempted to remove noise generated in MRI image acquisition by a non-invasive method different from a common contrast agent, thereby confirming that metal noise can be effectively removed by attaching a material including a functional group capable of hydrogen bonding to one region of a human body requiring diagnosis or by a method of covering the region with a pad including the material, and also confirming that the same effect can be obtained when the composition or pad used in the present invention is attached to or inserted into an MRI coil. Thus, the present invention was completed.

US2011/0152670A1 discloses a method of using high dielectric constant materials for reducing signal to noise ratio in MRI. Teeuwisse et al, Proc. Intl. Soc. Mag. Reson. Med 23 (2015) discloses barium titanate based dielectric materials for high field imaging. Zhang et al, Proc. Intl. Soc. Mag. Reson. Med 23 (2015) discusses signal to noise ratio performance of using dielectric pads with multiple channel RF coils.

B. Afsharipour et al, 2016 38th Annual International Conference of the IEEE Engineering in Medicine and Biology Society (EMBC), Orlando, FL, USA, 2016, pp. 3757-3760, doi: 10.1109/EMBC.2016.7591545, discloses using surface electromyography to detect changes in innervation zones pattern after human cervical spinal cord injury.

A. E. Franke et, Journal of Microelectromechanical Systems, vol. 12, no. 2, pp. 160-171, April 2003, doi: 10.1109/JMEMS.2002.805051, discloses polycrystalline silicon-germanium films for integrated microsystems.

Sejal Patel et al, Drug Development and Industrial Pharmacy, 36:4 (2010), pp. 379-384, DOI: 10.3109/03639040903188463, discloses Pharmacokinetics of amitriptyline in rabbit skin and plasma following iontophoretic administrations.

US 7 015 099 B2 discloses a test strip for determining dialysate composition.

### [Disclosure]

### [Technical Problems]

Therefore, to solve the above-described problems, the inventors have intensively attempted to remove noise when an MRI image is obtained, and thereby confirmed that noise caused by a metal material can be effectively removed and image quality can be improved by restoring noise in MRI images and phantom images using a material having a functional group capable of hydrogen bonding. Thus, the present invention was completed.

Accordingly, the present invention is directed to a method for removing noise from an MRI image using a pad comprising a composition which includes one or more compounds having a functional group capable of hydrogen bonding selected from the group consisting of: ethanol (C₂H₅OH), acetic acid (CH₃COOH), citric acid (C₆H₈O₇H₂O), sodium hydrogen carbonate (NaHCO₃) and glycerin (C₃H₈O₃).

The invention is also directed to the use of the pad for removing noise from an MRI image.

However, technical problems to be solved in the present invention are not limited to the above-described problems, and other problems which are not described herein will be fully understood by those of ordinary skill in the art from the following description.

### [Technical Solutions]

The composition for removing noise from an MRI image, used in the invention includes one or more compounds having a functional group capable of hydrogen bonding selected from the group consisting of: ethanol (C₂H₅OH), acetic acid (CH₃COOH), citric acid (C₆H₈O₇H₂O), sodium hydrogen carbonate (NaHCO₃) and glycerin (C₃H₈O₃).

The composition may improve the quality of an MRI image.

In addition, the water may be any one selected from the group consisting of light water, heavy water, tritiated water, and distilled water.

In addition, the compound having the hydrogen bond may be contained at 40 wt% or more with respect to the total weight of the composition, but the present invention is not limited thereto.

In addition, the composition used in the present invention may further include a material that can be used as a contrast agent to enhance an image, and the material that can be used as a contrast agent may be selected from the group consisting of gadoxetic acid disodium, calcium oxide, trometamol, sodium hydroxide, hydrochloric acid, an injection solution, calteridol calcium, gadolinium oxide, calcium, meglumine, EOB DTPA, gadoteridol and a chelating agent, but the present invention is not limited thereto.

In addition, the noise may be generated by a metal inserted into the body.

In addition, the composition used in the present invention is charged in a pad, and not only may the composition of the present invention be used as a pad for covering the body, but it may also be attached/detached to a main body coil, a shim coil, an RF coil, an array coil, a surface coil, a spine coil, a head coil, an extremity coil or a torso coil for an MRI apparatus.

In addition, the composition disclosed herein may be used in the manner of being injected into a main body coil, a shim coil, an RF coil, an array coil, a surface coil, a spine coil, a head coil, an extremity coil or a torso coil for an MRI apparatus.

The present invention uses a pad for removing noise from an MRI image, which includes the composition for removing noise from an MRI image.

The pad may improve the quality of MRI images, but the present invention is not limited thereto.

The pad used in the present invention may further include a cover accommodating the composition. The cover may be a vinyl cover, but the present invention is not limited thereto.

In addition, the pad used in the present invention may be a pad that can be transformed into a shape of the human body, for example, the head, limbs, spine or abdomen.

In addition, the pad used in the present invention may be a pad capable of being attached/detached to a main body coil, a shim coil, an RF coil, an array coil, a surface coil, a spine coil, a head coil, an extremity coil or a torso coil for an MRI apparatus, but the present invention is not limited thereto.

In addition, the pad may further include a filler, which may be a liquid gel form or a solid form. When the filler is a solid form, it may be a low-fluidic bead or powder type, or a combination of beads and a gel.

The composition for removing noise from an MRI image may be injected into a coil for an MRI apparatus.

The coil for an MRI apparatus may be one or more types of coils selected from the group consisting of a main body coil, a shim coil, an RF coil, an array coil, a surface coil, a spine coil, a head coil, an extremity coil, and a torso coil.

The composition including the compound having a functional group capable of hydrogen bonding may be used for removing noise from an MRI image.

An MRI image reduced in noise may be obtained by a method which includes: manufacturing a pad including a compound having a functional group capable of hydrogen bonding; attaching the pad to a desired region of a subject; and obtaining an MRI image in the desired region using an MRI apparatus.

The compound having a functional group capable of hydrogen bonding may be used for removing noise from an MRI image.

### [Advantageous Effects]

A compound having a functional group capable of hydrogen bonding disclosed herein has advantages of naturally generating a high signal through indirect energy exchange according to the magnetization of a hydrogen bond, and activating an image of tissue which is not properly embodied due to noise generated by a metal. A damaged tissue image due to a metallic material is theoretically impossible to reproduce and recover, but a pad as used in the present invention makes capturing the defected image possible and thus the image defect problem can be fundamentally solved. According to imaging technology, an image that is theoretically difficult to recover can be reproduced realistically. Also, the present invention is completed based on fundamental and concrete scientific knowledge, for example, a basic theory of MRI for obtaining a basic image, in which an organ of the human body is visualized according to the magnetic susceptibility of hydrogen, demonstrating an effect of removing noise caused by a metallic material and the improvement in image quality based on this theory.

In addition, since the composition used in the present invention can be applied by a non-invasive method, unlike an MRI contrast agent, it has no problem causing a side effect in the human body, no toxicity and this is safe. Moreover, the composition used in the present invention is used in the manner of covering a region in need of diagnosis or being attached to an MRI apparatus, such that there are advantages of improving a relatively wide range of images, and obtaining more accurate image data than that obtained when distorted images are restored by software using an algorithm. At the same time, the composition used in the present invention has a low price, such that it can be used as an effective and economical auxiliary means for improving an MRI image.

### [Description of Drawings]

FIG. 1A shows fruit juice acetic acid, a thickening agent and a glycerin (ultrasound gel) solution for manufacturing an acetic acid pad, and FIG. 1B shows a gel prepared by mixing the above-described materials.
FIG. 2 shows an acetic acid gel pad manufactured by containing mixed gel-type fruit juice acetic acid in a sanitary vinyl-type container.
FIG. 3 shows a phantom manufactured by adding a clip to a fat container.
FIG. 4A shows the result of comparing ROI mean values of a general phantom image, a water pad and an acetic acid pad, and FIG. 4B shows the comparison experiment result for a decrease in noise caused by a metallic material, showing the difference between a GE Healthcare auxiliary pad (left panel of FIG. 4B) and an acetic acid pad (right panel of FIG. 4B).
FIGS. 5A and 5B show the result of comparing an image with noise caused by a metallic material before (left panels of FIGS. 5A and 5B) and after (right panels of FIGS. 5A and 5B) the use of a pad, FIG. 5C shows the result of comparing phantom images according to the difference in magnetic susceptibility of the human body, and FIGS. 5D to 5L show the results of visually observing the improvement in noise caused by a metallic material and an image quality-improving effect before (left panels of FIGS. 5D to 5L) and after (right panels of FIGS. 5D to 5L) the use of a pad.
FIG. 6 shows the result of confirming the effect of removing noise from an MRI image using water and acetic acid.
FIG. 7 shows the result of confirming the effect of removing noise from an MRI image using citric acid.
FIG. 8 shows the result of confirming the effect of removing noise from an MRI image using sodium hydrogen carbonate.
FIG. 9 shows the result of confirming the effect of removing noise from an MRI image using ethanol.
FIG. 10 shows the result of confirming the effect of removing noise from an MRI image using glycerin.

### [Modes of the Invention]

### [Example 1] Preparation and methods of experiment

### 1-1. Manufacture of the acetic acid pad

Since acetic acid is easy to purchase and harmless to the human body and to investigate whether the removal of noise from an MRI image and an image quality-improving effect can be obtained using the acetic acid, a pad was manufactured using a naturally obtained concentration of acetic acid prepared from fruit juice.

A small amount of glycerin was mixed with fruit juice acetic acid, and a thickening agent was used to easily attach the mixture to a patient's implant site. Here, the used thickening agent refers to a mixture of glycerin (ultrasound gel component) and a common thickening component, and the thickening agent can be replaced with an ultrasound gel.

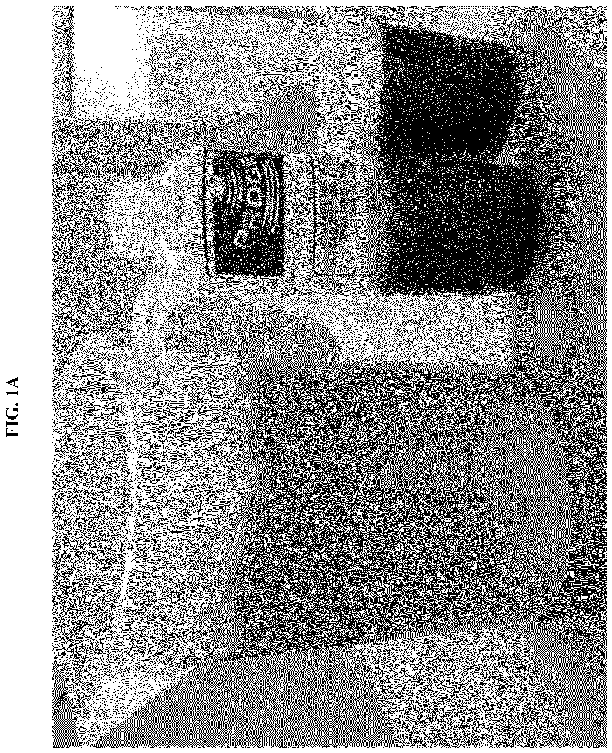

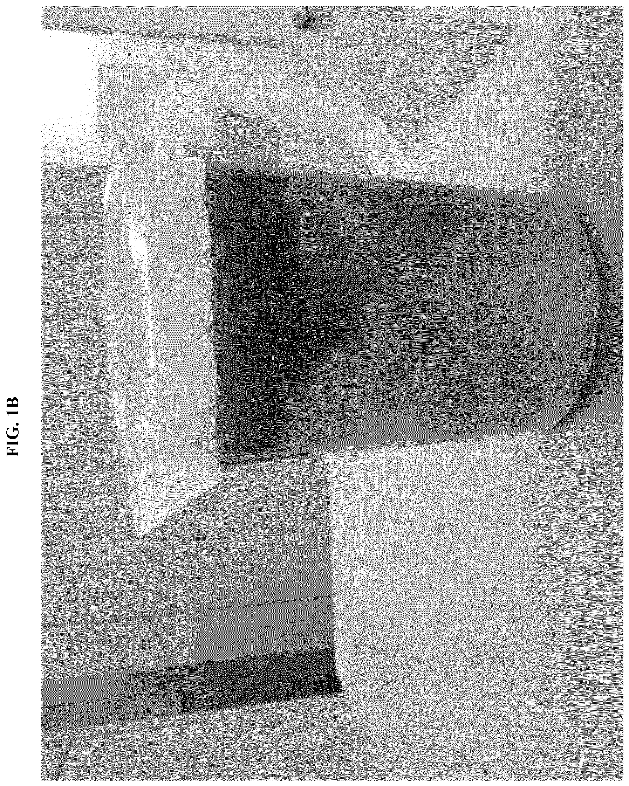

As shown in FIG. 1A, about 1 L of fruit juice acetic acid, a thickening agent and a glycerin (ultrasound gel) solution were prepared, and then they are mixed in a ratio of 200 mL : 800 mL (the fruit juice acetic acid: a mixture of a thickening agent and glycerin (ultrasound gel)) until the mixture becomes viscous, thereby preparing a gel (see FIG. 1B).

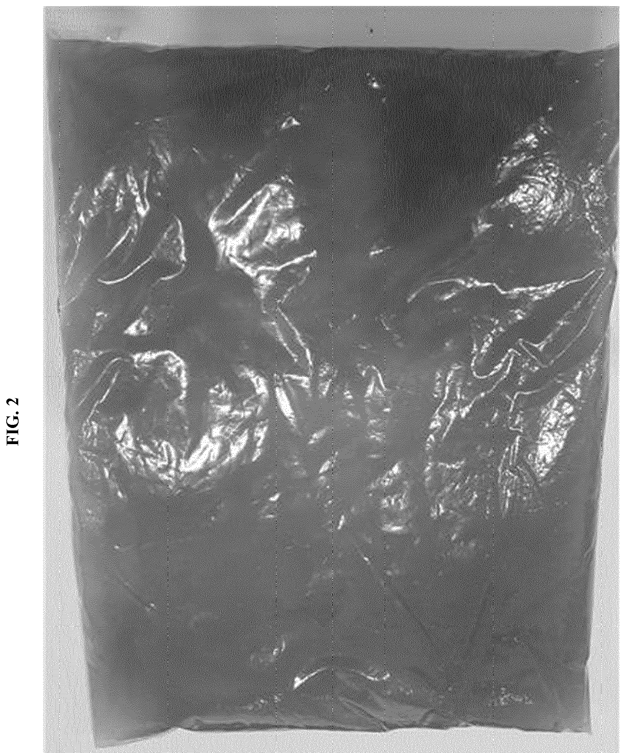

Afterward, as shown in FIG. 2, the mixed gel-type fruit juice acetic acid was contained in a sanitary vinyl-type container and stored at room temperature (15 to 25 °C), and then a test was performed once by attaching the acetic acid gel pad to the periphery of the implant at the final stage of the test for a patient who has given verbal consent.

### 1-2. Comparison experiment before/after use of acetic acid pad

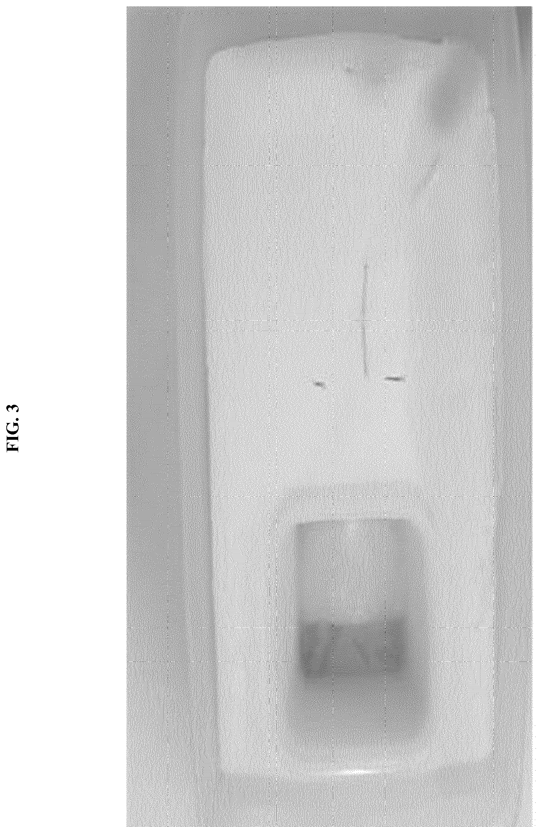

To overcome the limits of a human body experiment, as shown in FIG. 3, using a phantom manufactured by adding a clip to a fat container, a comparison experiment was sequentially performed on various pads, for example, a common phantom, a motion-preventing pad, a sandbag pad, a rice pad (tissue equivalent), a GE Healthcare abdominal fat-reducing pad, water, and an acetic acid pad.

More specifically, using specified protocol T1 Fat Saturation (Fov 240×207, TR 750ms, TE 12ms, Thickness 4mm, Gap 1.5mm), an international standard phantom was used as a target, and observation was performed before and after the use of a fruit juice acetic acid component pad. After the test, images of the region of interest (ROI) sites were obtained using MacroView (INFINITT) and a workstation ROI (contrast HU-value; M-view v 5.4.10.61).

After the phantom experiment, a patient observation test was performed before and after the use of the acetic acid pad, and thus images were obtained.

### [Example 2] Confirmation of experiment results for phantoms before/after use of acetic acid pad

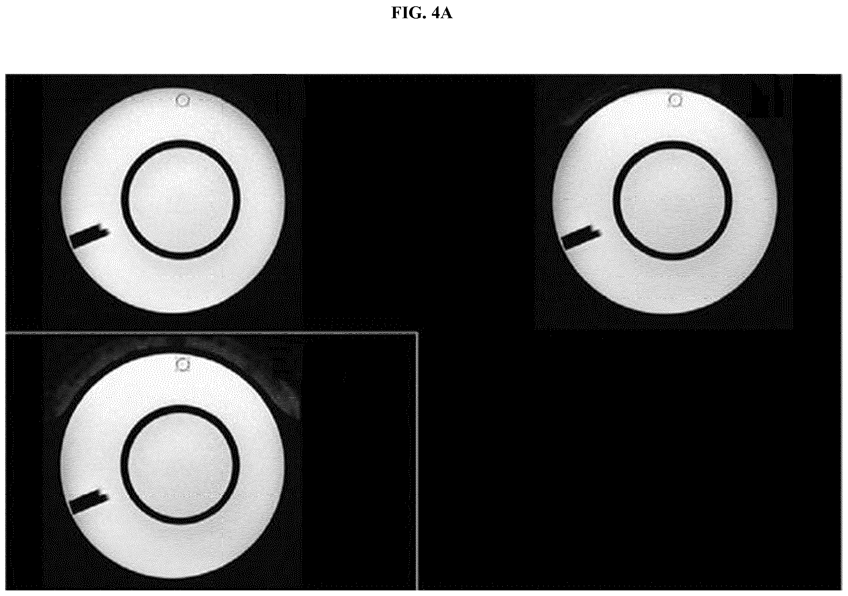

For international standard quality control (QC), that is, checking of the improvement of apparatus quality, an experiment was performed using the international standard QC phantom according to the method described in Example 1-2, and then a common phantom image, and the ROI mean values of a water pad and an acetic acid pad were compared. As a result, as shown in FIG. 4A and Table 1, the mean value of the non-mounted common phantom image was measured at 2029 (upper left panel of FIG. 4A), the mean value of the mounted water pad was measured at 2318 (the upper right panel of FIG. 4A), and the mean value of the acetic acid pad was measured at 2364 (lower panel of FIG. 4A).

**[Table 1]**

| Type | ROI mean value |
|---|---|
| Common phantom | 2029 |
| Water pad | 2318 |
| Acetic acid pad | 2364 |

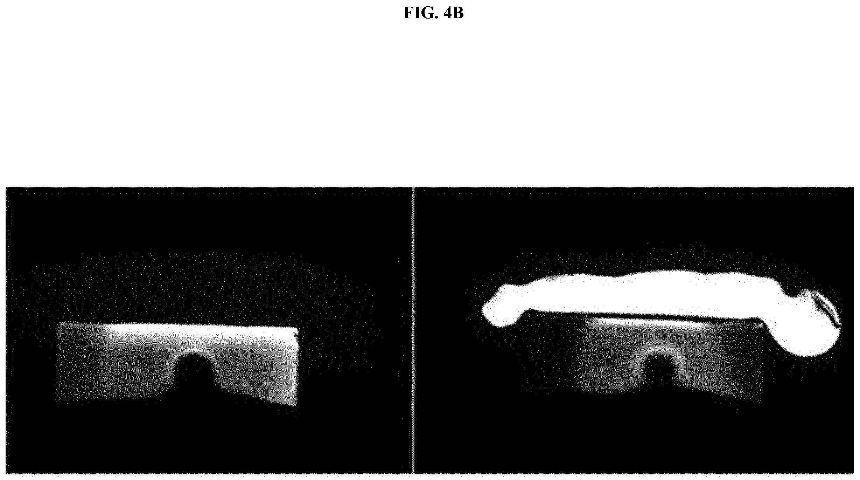

In addition, as shown in FIG. 4B, the comparison experiment for the reduction in noise caused by a metallic material showed a difference in images of the GE Healthcare auxiliary pad (left panel of FIG. 4B) and the acetic acid pad (right panel of FIG. 4B). From the above result, it can be confirmed that the higher the acetic acid content, the higher the ROI mean value, and thus the noise reduction rate was increased. Accordingly, it can be seen that acetic acid reduces the noise caused by a metallic material, demonstrating that the higher the hydrogen content and the higher the concentration of hydrogen, the higher the ROI measured value and noise-reducing effect.

### [Example 3] Confirmation of experiment results for patients before/after use of acetic acid pad

To compare the images with noise caused by a metallic material before and after the use of an acetic acid pad, patients were observed according to the method described in Example 1-2. Here, to compare the objective measurement data on the effect of using an acetic acid pad, the ROI mean value of the minimum and maximum standard deviations in an image quality contrast was compared.

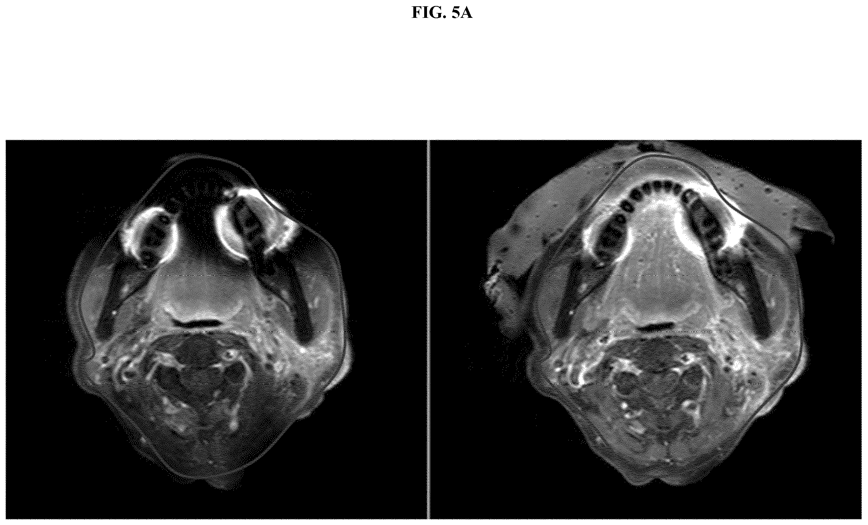

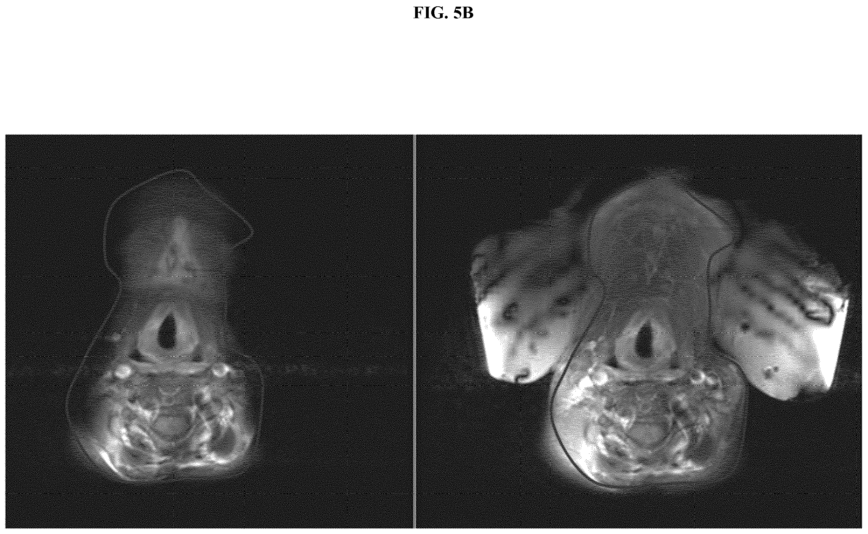

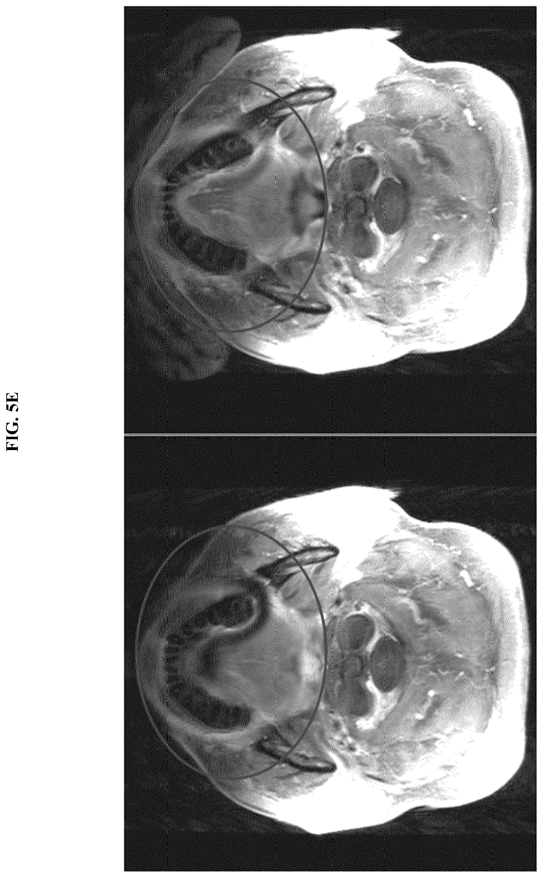

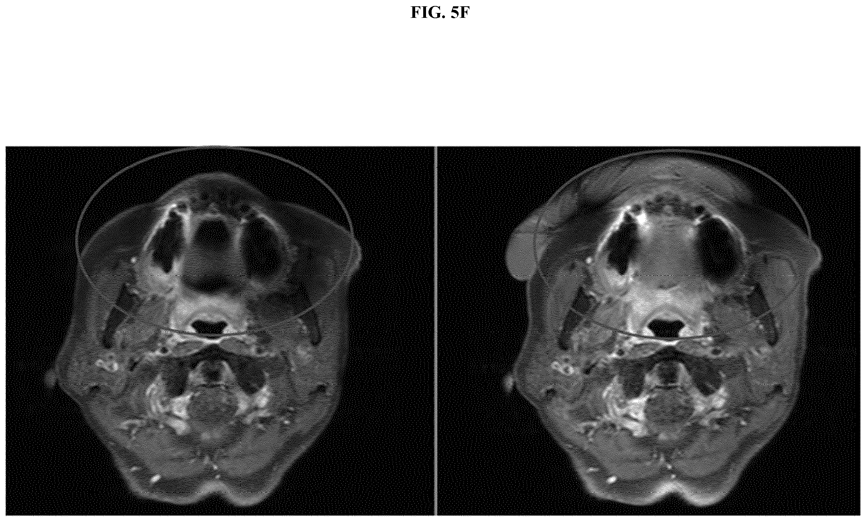

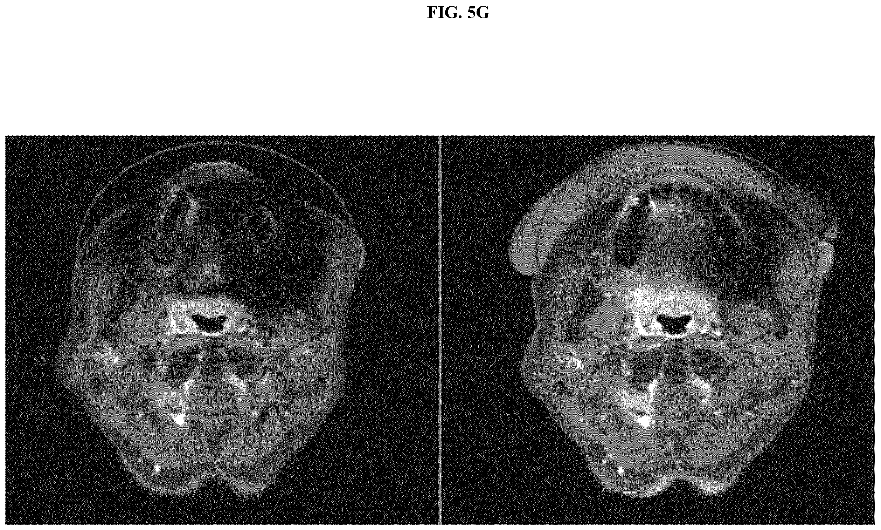

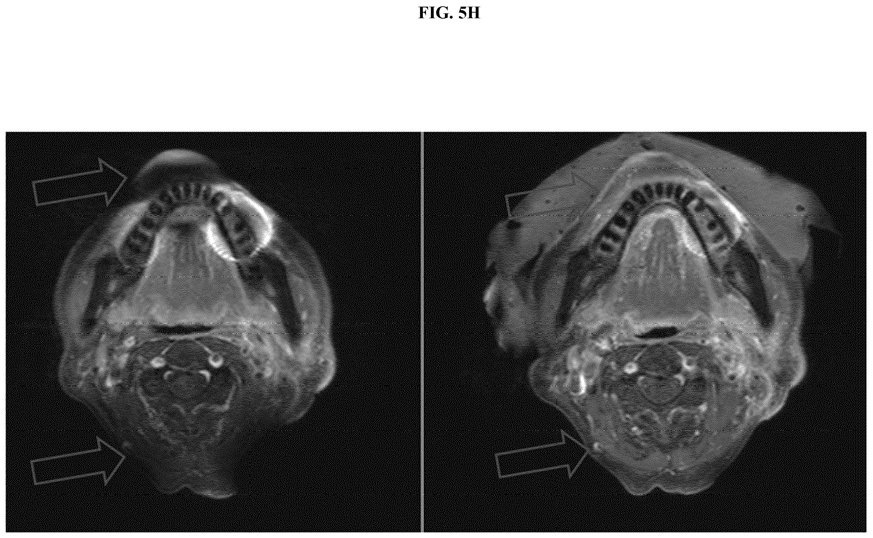

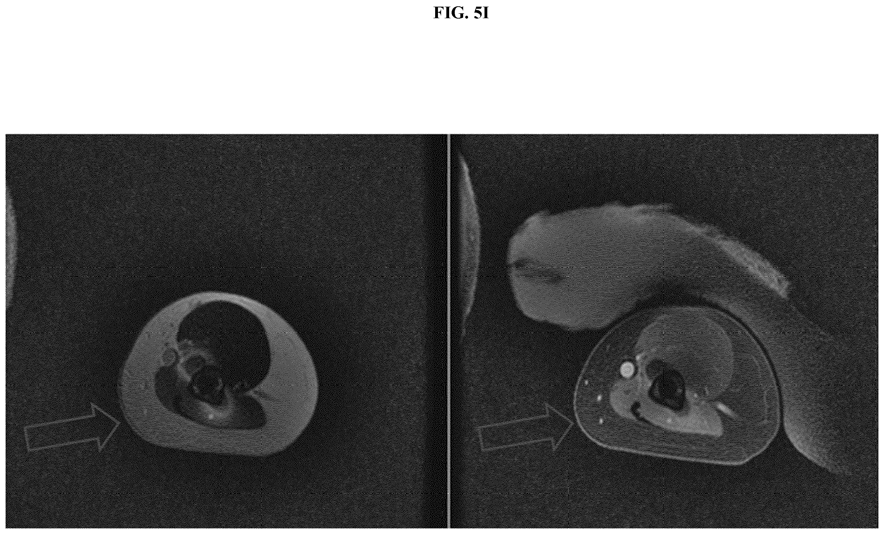

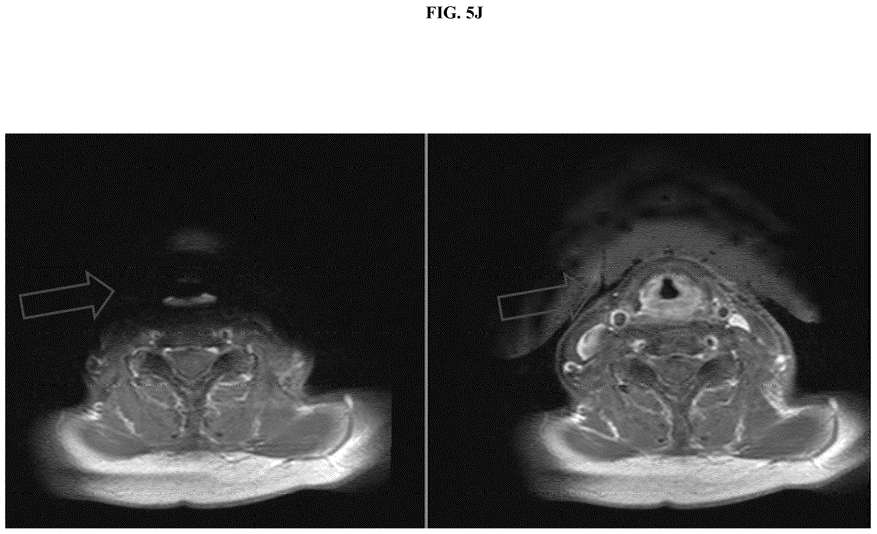

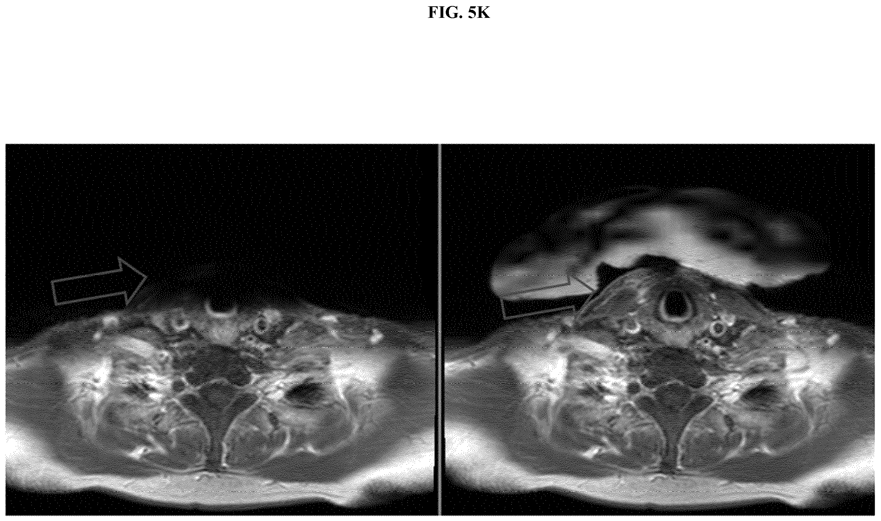

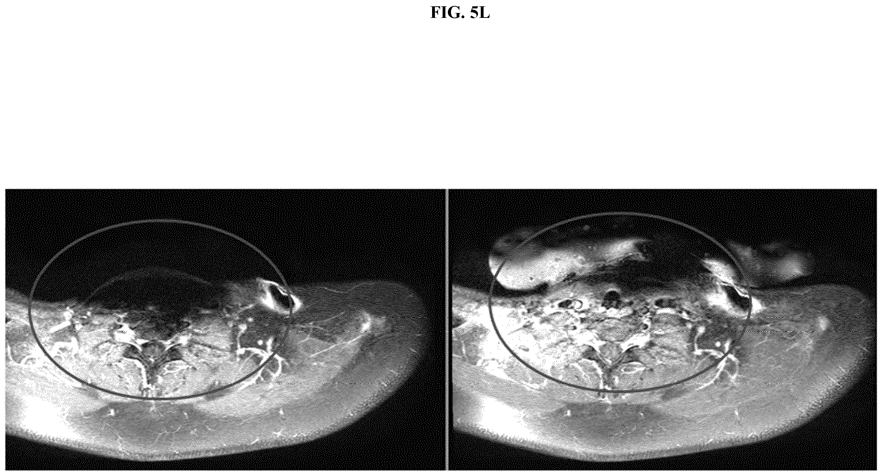

As a result, as shown in FIGS. 5A to 5C and Table 2, comparing the objective ROI mean values before and after the use of an acetic acid pad, the ROI mean value after the use of the pad (right panels of FIGS. 5A to 5C), compared with before the use of the pad (left panels of FIGS. 5A to 5C), were all increased, demonstrating that the image contrast after the use of an acetic acid pad is superior.

**[Table 2]**

| Drawing | ROI mean value | |
|---|---|---|
| | Before pad use (left panel of a drawing) | After pad use (right panel of a drawing) |
| FIG. 5A | 380 | 533 |
| FIG. 5B | 462 | 599 |
| FIG. 5C | 852 | 1011 |

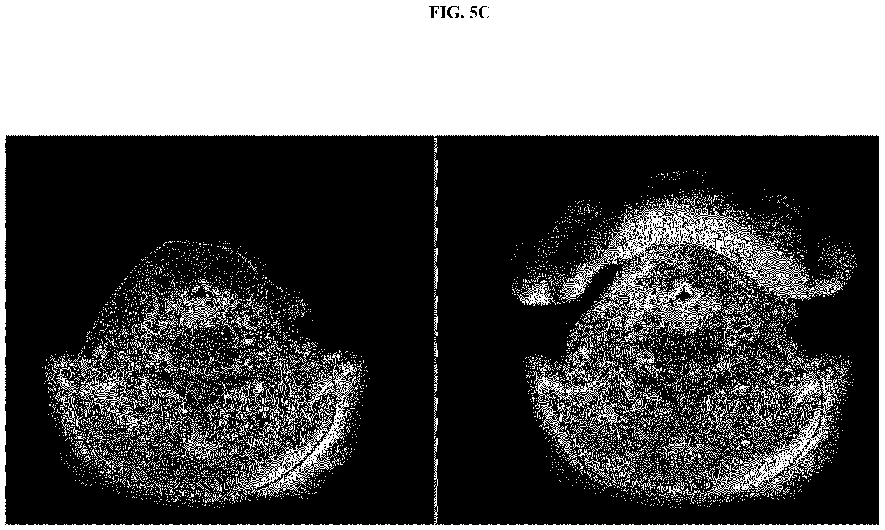

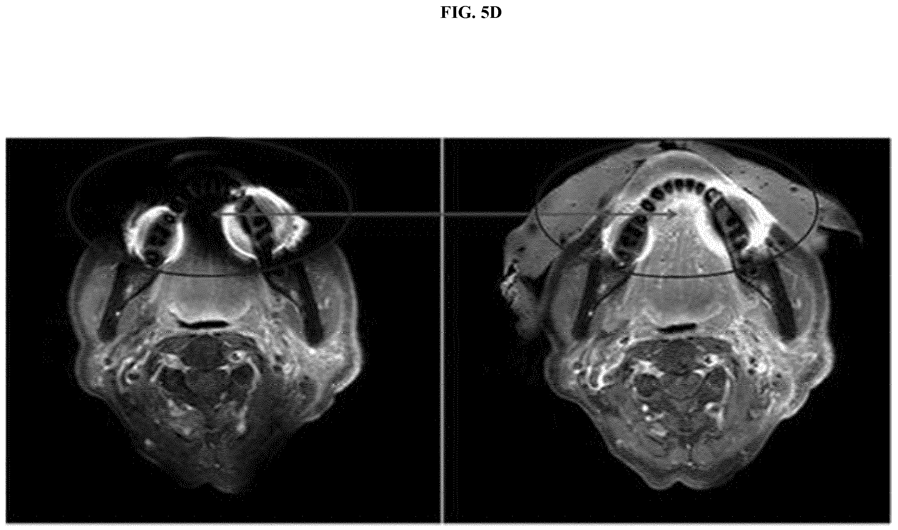

Moreover, FIG. 5C shows the result of comparing phantom images according to the difference in magnetic susceptibility of the human body, illustrating that the use of acetic acid pads (right panel of FIG. 5C) in an area of the cancer lesion of the neck, which is not shown in the phantom image (left panel of FIG. 5C), greatly improved the image quality. In addition, as shown in FIGS. 5D to 5L, since visual observation can show that the improvement in noise caused by a metallic material and the image quality improvement effect are obvious after the pad use (right panels of FIGS. 5D to 5L) compared with that before the pad use (left panels of FIGS. 5D to 5L), in MRI images, it can be deduced that the information of the MRI diagnosed region can be very broad using an acetic acid pad.

### [Example 4] Confirmation of the effect of removing noise from MRI image of the pad including a compound having a functional group capable of hydrogen bonding

As shown in Example 3, human images were obtained before and after the use of a pad consisting of acetic acid as a component, and to confirm the MRI image noise-removing effect of each pad filled with citric acid, sodium hydrogen carbonate, ethanol or glycerin, which has a high hydrogen content, for example, acetic acid, a region of interest (ROI) mean value in the image was measured using the phantom manufactured herein.

An acetic acid, citric acid, sodium hydrogen carbonate, ethanol or glycerin concentration of 0% represents water (H₂O), and an MRI image was obtained by targeting a component containing hydrogen, as a criterion for image acquisition, the natural state, water (H₂O), has been standardized according to MRI periodic inspection and international standard inspection.

### 4-1. Confirmation of removal of noise from MRI image using water (H₂O) and acetic acid

According to the method described in Example 1-1, a pad was manufactured by filling a pad with acetic acid (CH₃COOH), and an MRI image was measured.

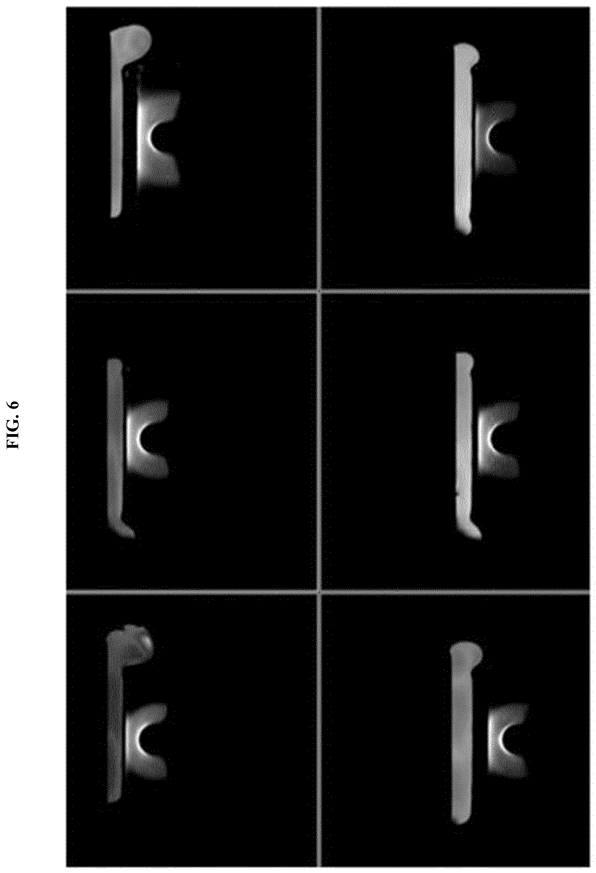

As a result, as shown in FIG. 6 and Table 3, it can be observed that, from the left side to the right side of FIG. 6 (the concentration of acetic acid in the upper panels was sequentially increased to 20% and 40% from 0% (H₂O), and similarly, the concentration of acetic acid in the lower panels was sequentially increased to 60%, 80% and 100% from the left side to the right side of the drawing), as the concentration of acetic acid was gradually increased, the ROI mean value was increased, and the noise reduction rate of an MRI image was increased.

**[Table 3]**

| Concentration of acetic acid (CH₃COOH) | ROI mean value (Mean) |
|---|---|
| 0% (H₂O)* | 1119 |
| 20% | 1147 |
| 40% | 1798 |
| 60% | 1868 |
| 80% | 1935 |
| 100% | 2004 |

| | |
|---|---|
| *reference example | |

### 4-2. Confirmation of removal of noise from MRI image using citric acid

In addition, according to the method described in Example 1-1, a pad was manufactured by filling a pad with citric acid (C₆H₈O₇H₂O), and an MRI image was measured.

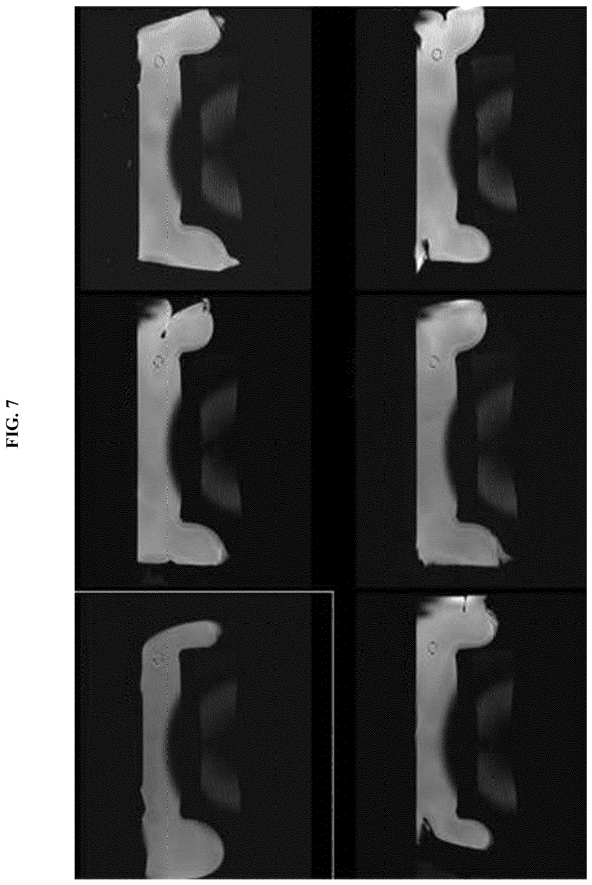

As a result, as shown in FIG. 7 and Table 4, it can be observed that, from the left side to the right side of FIG. 7 (the concentration of citric acid in the upper panels was sequentially increased to 20% and 40% from 0% (H₂O), and similarly, the concentration of citric acid in the lower panels was sequentially increased to 60%, 80% and 100% from the left side to the right side of the drawing), as the concentration of citric acid was gradually increased, the ROI mean value was increased, and the noise reduction rate of an MRI image was increased.

**[Table 4]**

| Concentration of citric acid (C₆H₈O₇H₂O) | ROI mean value (Mean) |
|---|---|
| 0% (H₂O)* | 1480 |
| 20% | 1698 |
| 40% | 1702 |
| 60% | 1734 |
| 80% | 1762 |
| 100% | 1797 |

| | |
|---|---|
| *reference example | |

### 4-3. Confirmation of removal of noise from MRI image using sodium hydrogen carbonate

According to the method described in Example 1-1, a pad was manufactured by filling a pad with sodium hydrogen carbonate (NaHCO₃), and an MRI image was measured.

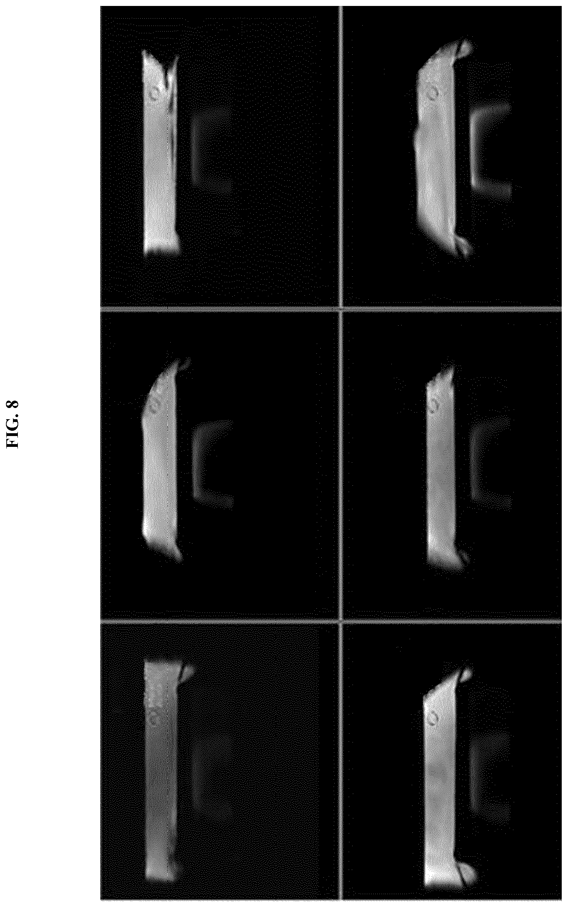

As a result, as shown in FIG. 8 and Table 5, it can be observed that, from the left side to the right side of FIG. 8 (the concentration of sodium hydrogen carbonate in the upper panels was sequentially increased to 20% and 40% from 0% (H₂O), and similarly, the concentration of sodium hydrogen carbonate at the lower panels was sequentially increased to 60%, 80% and 100% from the left side to the right side of the drawing), as the concentration of sodium hydrogen carbonate was gradually increased, the ROI mean value was increased, and the noise reduction rate of an MRI image was increased.

**[Table 5]**

| Concentration of sodium hydrogen carbonate (NaHCO₃) | ROI mean value (Mean) |
|---|---|
| 0% (H₂O)* | 1216 |
| 20% | 1439 |
| 40% | 1653 |
| 60% | 1622 |
| 80% | 1734 |
| 100% | 1730 |

| | |
|---|---|
| *reference example | |

### 4-4. Confirmation of removal of noise from MRI image using ethanol

According to the method described in Example 1-1, a pad was manufactured by filling a pad with ethanol (C₂H₅OH), and an MRI image was measured.

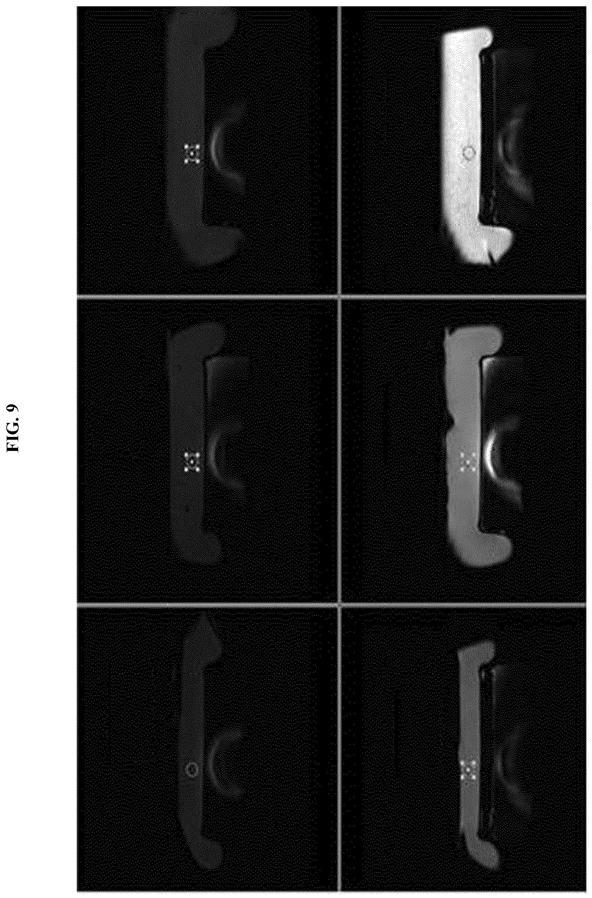

As a result, as shown in FIG. 9 and Table 6, it can be observed that, from the left side to the right side of FIG. 9 (the concentration of ethanol in the upper panels was sequentially increased to 20% and 40% from 0% (H₂O), and similarly, the concentration of ethanol in the lower panels was sequentially increased to 60%, 80% and 100% from the left side to the right side of the drawing), as the concentration of ethanol was gradually increased, the ROI mean value was increased, and the noise reduction rate of an MRI image was increased.

**[Table 6]**

| Concentration of ethanol (C₂H₅OH) | ROI mean value (Mean) |
|---|---|
| 0% (H₂O)* | 1519 |
| 20% | 1636 |
| 40% | 2168 |
| 60% | 5691 |
| 80% | 8655 |
| 100% | 14979 |

| | |
|---|---|
| *reference example | |

### 4-5. Confirmation of removal of noise from MRI image using glycerin

According to the method described in Example 1-1, a pad was manufactured by filling a pad with glycerin (C₃H₈O₃), and an MRI image was measured.

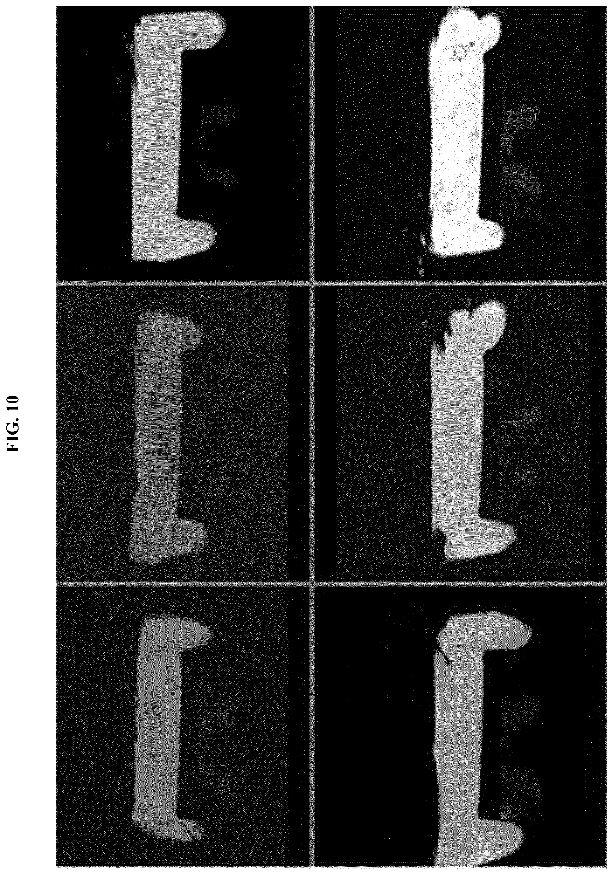

As a result, as shown in FIG. 10 and Table 7, it can be observed that, from the left side to the right side of FIG. 10 (the concentration of glycerin in the upper panels was sequentially increased to 20% and 40% from 0% (H₂O), and similarly, the concentration of glycerin in the lower panels was sequentially increased to 60%, 80% and 100% from the left side to the right side of the drawing), as the concentration of glycerin was gradually increased, the ROI mean value was increased, and the noise reduction rate of an MRI image was increased.

**[Table 7]**

| Concentration of glycerin (C₃H₈O₃) | ROI mean value (Mean) |
|---|---|
| 0% (H₂O)* | 1397 |
| 20% | 1488 |
| 40% | 1503 |
| 60% | 1538 |
| 80% | 1702 |
| 100% | 1712 |

| | |
|---|---|
| *reference example | |

### [Industrial Applicability]

The present invention relates to a method of removing MRI noise using a pad comprising one or more compounds having a functional group capable of hydrogen bonding as defined in the claims. The composition or pad reduces noise caused by a metal implant in the acquisition of MRI images, has an excellent image enhancement effect, is cheap and economical, has no toxicity in a human body and thus is safe. For this reason, the composition can be used as a component of an image diagnostic device in the field of medical devices, and is expected to be effectively used to acquire noise-removed MRI images in the field of diagnostic radiology.

## Claims

1. A method of removing noise from an MRI image using a pad comprising a composition comprising one or more compounds having a functional group capable of hydrogen bonding selected from the group consisting of: ethanol (C₂H₅OH), acetic acid (CH₃COOH), citric acid (C₆H₈O₇H₂O), sodium hydrogen carbonate (NaHCO₃) and glycerin (C₃H₈O₃).

2. The method according to claim 1, wherein the pad further comprises a cover accommodating the composition.

3. The method according to claim 1 or claim 2, wherein the pad is transformable into a shape of the human body.

4. The method according to any one of claims 1 to 3, wherein the pad is attachable/detachable to a main body coil, a shim coil, an RF coil, an array coil, a surface coil, a spine coil, a head coil, an extremity coil or a torso coil for an MRI apparatus.

5. A use of a pad for removing noise from an MRI image, the pad comprising a composition comprising one or more compounds having a functional group capable of hydrogen bonding selected from the group consisting of: ethanol (C₂H₅OH), acetic acid (CH₃COOH), citric acid (C₆H₈O₇H₂O), sodium hydrogen carbonate (NaHCO₃) and glycerin (C₃H₈O₃).

6. The use according to claim 5, wherein the pad further comprises a cover accommodating the composition.

7. The use according to claim 5 or claim 6, wherein the pad is transformable into a shape of the human body.

8. The use according to any one of claims 5 to 7, wherein the pad is attachable/detachable to a main body coil, a shim coil, an RF coil, an array coil, a surface coil, a spine coil, a head coil, an extremity coil or a torso coil for an MRI apparatus.

## Patentansprüche

1. Verfahren zum Entfernen von Rauschen aus einem MRT-Bild unter Verwendung eines Pads, das eine Zusammensetzung umfasst, die eine oder mehrere Verbindungen mit einer zur Wasserstoffbrückenbindung fähigen funktionellen Gruppe umfasst, ausgewählt aus der Gruppe bestehend aus: Ethanol (C₂H₅OH), Essigsäure (CH₃COOH), Zitronensäure (C₆H₈O₇H₂O), Natriumhydrogencarbonat (NaHCO₃) und Glycerin (C₃H₈O₃).

2. Verfahren nach Anspruch 1, wobei das Pad weiter eine Abdeckung umfasst, die die Zusammensetzung aufnimmt.

3. Verfahren nach Anspruch 1 oder Anspruch 2, wobei das Pad in eine Form des menschlichen Körpers transformierbar ist.

4. Verfahren nach einem der Ansprüche 1 bis 3, wobei das Pad an einer Hauptkörperspule, einer Shim-Spule, einer HF-Spule, einer Array-Spule, einer Oberflächenspule, einer Wirbelsäulenspule, einer Kopfspule, einer Extremitätenspule oder einer Torsospule für ein MRT-Gerät anbringbar/abnehmbar ist.

5. Verwendung eines Pads zum Entfernen von Rauschen aus einem MRT-Bild, das Pad umfassend eine Zusammensetzung, die eine oder mehrere Verbindungen mit einer zur Wasserstoffbrückenbindung fähigen funktionellen Gruppe umfasst, ausgewählt aus der Gruppe bestehend aus: Ethanol (C₂H₅OH), Essigsäure (CH₃COOH), Zitronensäure (C₆H₈O₇H₂O), Natriumhydrogencarbonat (NaHCO₃) und Glycerin (C₃H₈O₃).

6. Verwendung nach Anspruch 5, wobei das Pad weiter eine Abdeckung umfasst, die die Zusammensetzung aufnimmt.

7. Verwendung nach Anspruch 5 oder Anspruch 6, wobei das Pad in eine Form des menschlichen Körpers transformierbar ist.

8. Verwendung nach einem der Ansprüche 5 bis 7, wobei das Pad an einer Hauptkörperspule, einer Shim-Spule, einer HF-Spule, einer Array-Spule, einer Oberflächenspule, einer Wirbelsäulenspule, einer Kopfspule, einer Extremitätenspule oder einer Torsospule für ein MRT-Gerät anbringbar/abnehmbar ist.

## Revendications

1. Procédé d'élimination du bruit d'une image IRM en utilisant une pastille comprenant une composition comprenant un ou plusieurs composés présentant un groupe fonctionnel apte à la liaison hydrogène sélectionné dans le groupe consistant en : éthanol (C₂H₅OH), acide acétique (CH₃COOH), acide citrique (C₆H₈O₇H₂O), hydrogénocarbonate de sodium (NaHCO₃) et glycérine (C₃H₅O₃).

2. Procédé selon la revendication 1, dans lequel la pastille comprend en outre un couvercle accueillant la composition.

3. Procédé selon la revendication 1 ou la revendication 2, dans lequel la pastille est transformable en une forme du corps humain.

4. Procédé selon l'une quelconque des revendications 1 à 3, dans lequel la pastille est attachable/détachable à une bobine de corps principal, une bobine de cale, une bobine RF, une bobine de réseau, une bobine de surface, une bobine rachidienne, une bobine de tête, une bobine d'extrémité ou une bobine de torse pour un appareil d'IRM.

5. Utilisation d'une pastille pour éliminer le bruit d'une image IRM, la pastille comprenant une composition comprenant un ou plusieurs composés présentant un groupe fonctionnel apte à se lier à l'hydrogène sélectionné dans le groupe consistant en : éthanol (C₂H₅OH), acide acétique (CH₃COOH), acide citrique (C₆H₈O₇H₂O), hydrogénocarbonate de sodium (NaHCO₃) et glycérine (C₃H₈O₃).

6. Utilisation selon la revendication 5, dans laquelle la pastille comprend en outre un couvercle accueillant la composition.

7. Utilisation selon la revendication 5 ou la revendication 6, dans laquelle la pastille est transformable en une forme du corps humain.

8. Utilisation selon l'une quelconque des revendications 5 à 7, dans laquelle la pastille est attachable/détachable à une bobine de corps principal, une bobine de cale, une bobine RF, une bobine de réseau, une bobine de surface, une bobine rachidienne, une bobine de tête, une bobine d'extrémité ou une bobine de torse pour un appareil d'IRM.
